# EUROPÄISCHE PATENTSCHRIFT

(11) **EP 0 625 035 B1**
(45) Veröffentlichungstag und Bekanntmachung des Hinweises auf die Patenterteilung: **24.04.1996**
(21) Anmeldenummer: 92903615.0
(22) Anmeldetag: 03.02.1992
(51) Int. Cl.: A61F 13/02

(54) **VERFAHREN ZUR HERSTELLUNG MIT PORÖSEN KLEBSTOFFSCHICHTEN BESCHICHTETER FLEXIBLER, FLÄCHIGER TRÄGER**
DEVICE FOR PRODUCING FLEXIBLE, FLAT SUBSTRATES WITH POROUS ADHESIVE COATINGS
PROCEDE DE FABRICATION D'UN SUPPORT SOUPLE ET MINCE RECOUVERT DE COUCHES POREUSES D'ADHESIF

(43) Veröffentlichungstag der Anmeldung: 23.11.1994
(73) Patentinhaber: Beiersdorf Aktiengesellschaft, D-20245 Hamburg (DE)
(72) Erfinder: CARSTEN, Rolf, D-22309 Hamburg (DE); SCHULZE, Rolf, D-2423 Kasseedorf (DE); VON WOLFF, Axel, D-2100 Hamburg 90 (DE)
(86) Internationale Anmeldenummer: DE9200065
(87) Internationale Veröffentlichungsnummer: WO9314725

(56) Entgegenhaltungen:
- DE-A- 4 042 441

## Beschreibung

Die Erfindung betrifft Verfahren zur Beschichtung von Trägermaterialien mit Klebstoffen. Insbesondere betrifft sie Verfahren zur Beschichtung von flächigen Trägern wie z.B. Gewebebändern, Vliesen, Papier- oder Kunststoffbändern.

Mit Klebstoff beschichtete Träger, zumal flächige Träger, sind an sich bekannt und weit verbreitet. So sind mit Klebstoff beschichtete Kunststoff- und Papierbänder seit langer Zeit ein alltägliches Hilfsmittel in Büro und Haushalt. Selbstklebende Pflaster als Wundschnellverbände sind gleichfalls seit vielen Jahrzehnten in vielfältiger Ausfertigung dem Verbraucher ins Bewußtsein gedrungen.

In vielen Fällen bestehen die Träger aus nichtokklusivem Material, d.h., sie sind für Wasserdampf und/oder Luft weitgehend durchlässig. Dies ist in manchen Fällen erwünscht, denn ein Wundschnellverband beispielsweise ist in den weitaus meisten Fällen umso besser hautverträglich, je besser er für Luft und Feuchtigkeit durchlässig ist.

Um allerdings die Klebstoffbeschichtung nichtokklusiv zu gestalten, hat man sich in der Vergangenheit stets unbequemer, wenig praktikabler und zumeist höchst unökonomischer Verfahren bedienen müssen.

Es ist bekannt, den Träger mit einem flächenmäßig begrenzten, oder von klebstofffreien Stellen unterbrochenen Klebstoffauftrag zu versehen. Es ist auch bekannt, das mit der Klebstoffmasse beschichtete Material nachträglich, beispielsweise mit Hilfe von Luftdüsen, zu perforieren.

Diese Verfahren sind aber ungeeignet, mikroporöse Klebstoffschichten mit wirklich befriedigenden Eigenschaften zu erzeugen.

Weiterhin ist aus der DE-PS 15 69 901 das folgende Verfahren zur Herstellung poröser selbstklebender Bänder oder Blätter bekannt: Man trägt eine lösungsmittelhaltige viskoelastische Selbstklebemasse auf einen Zwischenträger mit klebstoffabweisender Oberfläche auf, erhöht die Temperatur schnell, so daß das Lösungs- oder Dispersionsmittel verdampft und die viskoelastische Selbstklebemasse Bläschen wirft. Der so beschichtete Zwischenträger wird, nachdem die Klebstoffschicht getrocknet ist, mit erhöhtem Druck gegen eine poröse Unterlage, einen flächigen Träger also, gepreßt. Die Selbstklebemasse wird so auf die Unterlage übertragen. Der erhöhte Druck bewirkt dabei, daß die Bläschen zerplatzen und feine Poren entstehen.

Die hierbei entstehenden Klebstoffschichten sind zwar mikroporös, es zerplatzen aber längst nicht alle Bläschen. In Bezug auf seine theoretisch zu erwartenden Ergebnisse wirkt also auch das hier beschriebene Verfahren nicht optimal.

Dazu kommt, daß der verwendete Papierträger nicht beliebig oft wiederverwendbar ist. Das Verfahren ist also teuer.

Die DE-OS 36 06 199 beschreibt einen Klebezettel mit einer Klebstoffschicht, deren Oberfläche innerhalb eines im wesentlichen vollflächig mit der Kleberschicht versehenen Bereiches Erhöhungen und Vertiefungen aufweist. Es werden keine porösen oder gar mikroporösen Klebstoffschichten beschrieben, sondern vollflächige Beschichtungen.

Die Oberflächenstrukturierung die in dieser Schrift beschrieben wird, ist makroskopischer Natur.

Durch diese Oberflächenstruktur werden weder Luft noch Lösungsmitteldämpfe von Zwischenträger und Klebstoffschicht eingeschlossen. Vielmehr wird nur erreicht, daß die Klebstoffschicht an der einen Stelle dicker und an der anderen Stelle dünner ist, wobei sie eine wellige Oberfläche annimmt.

Offensichtlich ist ferner, daß die hier konzipierten Selbstklebeobjekte fernab jeder medizinischer Verwendung sind, da sie sich nicht nur leicht vom Substrat abheben, sondern auch, weil sie nicht porös sind, das Substrat okkludieren.

Die DE-OS 27 19 779 betrifft einen selbstklebenden, porösen, luftdurchlässigen Streifen für die Verwendung als Verbandmaterial. Die Poren werden erzeugt, indem ein Treibmittel, welches in der Klebstoffmasse gelöst vorliegt, erhitzt wird und sich dadurch ausdehnt. Dabei sollen die so erzeugten Treibmittelbläschen die Klebstoffschicht zerreißen. Es werden in dieser Schrift Treibmittel beschrieben, auf die gemäß der vorliegenden Erfindung gänzlich verzichtet werden kann. Im einzigen Beispiel wird (NH₄)₂CO₃, welches auch Bestandteil des Backpulvers ist, als Treibmittel genannt. Die Gefahr, daß der bei der thermischen Zersetzung des (NH₄)₂CO₃ entstehende Ammoniak unerwünschte Nebenreaktionen, beispielweise mit der Klebmasse selbst, oder aber dem Träger eingeht, ist groß. Zudem muß das als Festkörper vorliegende (NH₄)₂CO₃ aufwendig vermahlen und in der Klebmasse gleichmäßig verteilt werden.

Außerdem ist zu erwarten, daß das (NH₄)₂CO₃ in der Klebmasse sedimentiert, also eben keine gleichmäßige Treibmittelverteilung erreicht wird. Sedimentation ist eine bei der Zerteilung pulveriger Festkörpern in Klebmassen eine wohlbekannte unangenehme Begleiterscheinung. Dabei entstehen Zonen hoher Festkörperanballung und solche mit niedrigem Festkörpergehalt.

Obzwar eine Druckbehandlung analog zur DE-AS 15 69 901 weder beschrieben noch beansprucht wird, weiß der Fachmann, daß das in der Klebmasse mehr oder minder gleichmäßig verteilte Treibmittel - neben gelegentlich auftretenden Poren - hauptsächlich geschlossene Blasen bildet. Diese müssen durch mechanische Einwirkung geöffnet werden.

Es war also Aufgabe der vorliegenden Erfindung, ein Verfahren zu entwickeln, mit Hilfe dessen auf ökonomische Weise mikroporöse Klebstoffschichten erhältlich sind, ohne daß das Verfahren oder die Produkte mit den Mängeln des Standes der Technik behaftet sind.

Erfindungsgemäß wird die Aufgabe gelöst durch ein Verfahren gemäß Anspruch 1.

Unter dem Begriff "mikroskopisch gewellte, gefältelte zerklüftete oder gefurchte Oberfläche" ist im Rahmen dieser Anmeldung zu verstehen, daß die Oberfläche des Zwischenträgers nicht glatt sein soll, sondern vielmehr unregelmäßige oder regelmäßige Erhebungen und/oder Vertiefungen aufweist. Diese Struktur soll unter licht- oder elektronenmikroskopischer Vergrößerung erkennbar sein. Besonders vorteilhaft ist, wenn die Oberfläche des Zwischenträgers im mikroskopischen Bild der Schale einer Apfelsine oder einer Hügellandschaft ähnelt.

Gemäß der vorliegenden Erfindung wird die Porosität dadurch erreicht, daß zwischen Klebstoffschicht und Zwischenträger befindliche Luft durch Ausdehnung die Klebstoffschicht durchbricht.

Durch die vorteilhaften Eigenschaften der vorliegenden Erfindung ist eine Nachbehandlung, nämlich die Ausübung zusätzlichen mechanischen Druckes auf den Träger, damit sich die Poren überhaupt erst bilden, gänzlich unnötig.

Es gilt erfindungsgemäß zu beachten, daß die Klebstoffmasse beim Auftragen die Vertiefungen des Zwischenträgers nicht gänzlich ausfüllen soll, sondern, daß zwischen der Klebstoffmasse und dem Zwischenträger Hohlräume entstehen, die mit Luft und/oder Lösungsmitteldampf gefüllt sind.

Dem Fachmann ist bekannt, wie die Viskosität von Klebstoffmassen beeinflußt werden kann und ist leicht imstande, die Viskosität der mikroskopisch gewellten, gefältelten, zerklüfteten oder gefurchten Oberfläche durch einfaches Probieren anzupassen.

Besonders vorteilhaft ist es, die Klebstoffmasse aus der Gruppe der üblichen Selbstklebemassen, insbesondere der druckempfindlichen Selbstklebemassen, zu wählen. Die Träger können vorteilhaft gewählt werden aus der Gruppe der Vliese, der Gewebe-, Papier- oder Kunststoffolien oder dergleichen.

Die Zwischenträger mit mikroskopisch gewellter, gefältelter, zerklüfteter oder gefurchter Oberfläche werden vorteilhaft durch Beschichten eines flexiblen Grundkörpers aus dauerhaftem Material mit einem polymeren Kunststoff erhalten. Es ist günstig, zuerst den Kunststoff aufzutragen, und dem Kunststoff vor dem Aushärten mit einem oberflächenstrukturierten Gegenstande, z.B. einem umlaufenden Schaumstoffband oder einer Schaumstoffrolle, eine erfindungsgemäße Oberflächenstruktur zu verleihen. Wird der Kunststoff zuerst aufgetragen und dann strukturiert, so sind grundsätzlich alle Beschichtungsverfahren, so zum Beispiel auch Sprühverfahren, einsetzbar.

Es ist aber auch möglich und vorteilhaft, die Beschichtung selbst mit einer oberflächenstrukturierten Vorrichtung, insbesondere einer saugfähigen, besonders bevorzugt einer porösen Vorrichtung, wie etwa einer Schaumstoffrolle, vorzunehmen. Die Oberfläche des Kunststoffes nimmt bei dieser Art der Beschichtung ohne weitere Behandlung die erfindungsgemäße Struktur an.

Als erfindungsgemäß werden demnach angesehen: ein Verfahren zur Herstellung eines Zwischenträgers nach Anspruch 1, dadurch erhältlich, daß ein in flüssiger Form vorliegender Kunststoff mittels einer oberflächenstrukturierten Vorrichtung, insbesondere einer saugfähigen, besonders bevorzugt einer porösen Vorrichtung, beispielsweise einer Schaumstoffrolle, auf einen flexiblen Grundkörper aufgetragen wird, sowie ein Verfahren zur Herstellung eines Zwischenträgers nach Anspruch 1, dadurch erhältlich, daß ein in flüssiger Form vorliegender Kunststoff auf einen flexiblen Grundkörper aufgetragen wird und dem Kunststoff vor dem Aushärten mit einer oberflächenstrukturierten Vorrichtung, insbesondere einer saugfähigen, besonders bevorzugt einer porösen Vorrichtung, beispielsweise einer Schaumstoffrolle oder einem umlaufenden Schaumstoffband, eine Oberflächenstruktur verliehen wird.

Günstig ist, den beschichteten und oberflächenstrukturierten Zwischenträger in einer Trocknungsanlage austrocknen zu lassen.

Der Grundkörper für die Zwischenträger kann aus allen gängigen Materialien für solche Zwecke gewählt werden. Besonders vorteilhaft sind gewebte Gurtbänder aus Glasfaser. Aber auch Gummitücher, Kunststoffbänder und dergleichen haben sich als günstig herausgestellt. Es ist, wenn Glasfaserbänder gewählt werden, günstig, solche zu verwenden, die schon mit einer im wesentlichen unstrukturierten Oberflächenbeschichtung aus Kunststoff versehen sind. Diese letztere Beschichtung fördert die Haftung der erfindungsgemäßen Kunststoffbeschichtung mit Oberflächenstruktur auf dem Grundkörper.

Die Kunststoffe, mit welchen die Zwischenträger beschichtet werden, können vorteilhaft gewählt werden aus der Gruppe der Siliconkautschuke, obwohl auch andere dauerhafte Kunststoffe durchaus in Betracht kommen. Diese Kunststoffe können mit den üblichen Beschichtungsverfahren, beispielsweise mittels Streichmesserbeschichtung, auf den Zwischenträger aufgebracht werden. Es ist vorteilhaft, die Kunststoffe in gelöster Form einzusetzen. Dennoch ist es gegebenenfalls vorteilhaft, die Kunststoffe, sofern sie selbst fließfähig sind, in ungelöster Form einzusetzen.

In den nachfolgenden Beispielen soll die vorliegende Erfindung näher erläutert werden, ohne daß aber die Erfindung auf diese Beispiele eingeschränkt werden soll. Vielmehr ist der Fachmann durch sein Fachwissen in der Lage, Modifikationen vorzunehmen, die den Bereich der vorliegenden Erfindung nicht verlassen.

### Beispiel 1

### Erzeugung eines oberflächenstrukturierten Zwischenträgers:

Ein gewebtes Glasfaserband mit transparenter Siliconbeschichtung (Beschichtungsdicke ca. 0,5 mm, Gesamtdicke ca. 2 mm) wird mittels Streichmesserverfahren mit Siliconkautschuk beschichtet:

Verwendet wird ein acetathärtender dauerelastischer Siliconkautschuk der Shorehärte > 25, Dichte 1,0 - 1,15 kg/m³ verwendet. Er wird mit Benzin 60/95 auf geeignete Viskosität verdünnt.

Diese Lösung wird mit einem Streichmesser auf das Glasfaserband aufgetragen. Kurz hinter dem Streichmesser wird die Oberfläche des Siliconkunststoffes mit einer Schaumstoffrolle nachbehandelt.

Das so beschichtete Glasfaserband wird mit einer Bahngeschwindigkeit von 1,5 m/min bei 100° C durch einen 9 m langen Düsentrockner gefahren. Nach endgültiger Trocknung (24 Stunden bei Raumtemperatur) ist der Zwischenträger einsatzbereit.

### Beispiel 2

### Beschichtung eines flexiblen Trägers mit Selbstklebemasse

Auf einem Zwischenträger gemäß Beispiel 1 wird nach dem sogenannten Tuchrakelverfahren mittels Rakelsystem eine viskoelastische Selbstklebemasse, die durch Copolymerisation von 490 Gewichtsteilen 2-Ethylhexylacrylat, 490 Gewichtsteilen n-Butylacrylat und 20 Gewichtsteilen Glycidylmethacrylat erhalten wurde, aus einer Lösung in einem Aceton-Benzin-Gemisch in einer Menge, entsprechend einer Schichtstärke von etwa 45 g/m² nach dem Trocknen, aufgetragen. Der nunmehr mit der Selbstklebemasse bedeckte Zwischenträger wird mit einer Geschwindigkeit von etwa 15 m/min durch einen stufenweise beheizbaren Trockenkanal geleitet der in sechs Heizzonen mit folgenden Temperaturen eingeteilt ist: 100/100/100/100/90/80° C.

Während der Trocknung dehnt sich die zwischen der Oberfläche des Zwischenträgers und der Selbstklebemasse befindliche Luft (bzw. das Lösungsmitteldampf/Luftgemisch) aus und läßt die Klebstoffschicht an den betreffenden Stellen aufplatzen.

Die nunmehr feinlöchrige Klebstoffschicht wird mittels Andrückwalzen auf ein Wirrfaservlies (Viscose, ca. 30 g/m²) kaschiert.

Das fertige, mikroporös beschichtete Wirrfaservlies kann in Streifen gewünschter Breite geschnitten und auf Rollen gewickelt werden. Um leichteres Abwickeln zu ermöglichen, kann es vorteilhaft sein, die Rückseite des eingesetzten Trägers (hier: des Wirrfaservlieses), welche nicht mit der Selbstklebemasse beschichtet wird, mit einer Schicht klebstoffabweisenden Materials zu bedecken.

### Beispiel 3

### Beschichtung einer perforierten Folie mit Selbstklebemasse

Auf einem Zwischenträger gemäß Beispiel 1 wird nach dem sogenannten Tuchrakelverfahren mittels Rakelsystem eine viskoelastische Selbstklebemasse, die durch Copolymerisation von 490 Gewichtsteilen 2-Ethylhexylacrylat, 496 Gewichtsteilen n-Butylacrylat und 20 Gewichtsteilen Glycidylmethacrylat erhalten wurde, aus einer Lösung in einem Aceton-Benzin-Gemisch in einer Menge, entsprechend einer Schichtstärke von etwa 45 g/m² nach dem Trocknen, aufgetragen. Der nunmehr mit der Selbstklebemasse bedeckte Zwischenträger wird mit einer Geschwindigkeit von etwa 15 m/min durch einen stufenweise beheizbaren Trockenkanal geleitet der in sechs Heizzonen mit folgenden Temperaturen eingeteilt ist: 100/100/100/100/90/80° C.

Während der Trocknung dehnt sich die zwischen der Oberfläche des Zwischenträgers und der Selbstklebemasse befindliche Luft aus und läßt die Klebstoffschicht an den betreffenden Stellen aufplatzen.

Die nunmehr feinlöchrige Klebstoffschicht wird mittels Andrückwalzen auf eine perforierte Folie kaschiert. Die fertige, mikroporös beschichtete Folie kann in Streifen gewünschter Breite geschnitten und auf Rollen gewickelt werden.

### Beispiel 4

### Beschichtung eines Gewebebandes mit Selbstklebemasse

Auf einem Zwischenträger gemäß Beispiel 1 wird nach dem sogenannten Tuchrakelverfahren mittels Rakelsystem eine viskoelastische Selbstklebemasse, die durch Copolymerisation von 490 Gewichtsteilen 2-Ethylhexylacrylat, 490 Gewichtsteilen n-Butylacrylat und 20 Gewichtsteilen Glycidylmethacrylat erhalten wurde, aus einer Lösung in einem einem Aceton-Benzin-Gemisch in einer Menge, entsprechend einer Schichtstärke von etwa 45 g/m² nach dem Trocknen, aufgetragen. Der nunmehr mit der Selbstklebemasse bedeckte Zwischenträger wird mit einer Geschwindigkeit von etwa 10 m/min durch einen stufenweise beheizbaren Trockenkanal geleitet der in sechs Heizzonen mit folgenden Temperaturen eingeteilt ist: 100/100/100/100/90/80° C.

Während der Trocknung dehnt sich die zwischen der Oberfläche des Zwischenträgers und der Selbstklebemasse befindliche Luft aus und läßt die Klebstoffschicht an den betreffenden Stellen aufplatzen.

Die nunmehr feinlöchrige Klebstoffschicht wird mittels Andrückwalzen auf ein elastisches Gewebe (ca. 300 g/m²) kaschiert. Das fertige, mikroporös beschichtete Gewebe kann in Streifen gewünschter Breite geschnitten und auf Rollen gewickelt werden.

## Patentansprüche

1. Verfahren zur Herstellung eines mit einer mikroporösen Klebstoffschicht beschichteten flexiblen, flächigen Trägers, dadurch gekennzeichnet, daß
a) eine fließfähige Klebstoffmasse auf einen Zwischenträger aufgetragen wird, der folgende Eigenschaften aufweist:
- er hat eine unter dem Licht- oder Elektronenmikroskop erkennbare gewellte, gefältelte, zerklüftete oder gefurchte Oberfläche
- die Klebstoffmasse ist von seiner Oberfläche leicht ablösbar
- er ist im wesentlichen luftundurchlässig,
wobei die Klebstoffmasse beim Auftragen die Vertiefungen des Zwischenträgers nicht gänzlich ausfüllt,
b) die nach der Beschichtung des Zwischenträgers zwischen der Klebstoffmasse und dem Zwischenträger entstehenden mikroskopischen Luft- oder Lösungsmitteleinschlüsse durch Temperaturerhöhung ausgedehnt werden, bis die Oberfläche der Klebstoffmasse aufplatzt, und
c) die Klebstoffmasse anschließend von dem Zwischenträger auf den endgültigen Träger übertragen wird.

2. Verfahren nach Anspruch 1, dadurch gekennzeichnet, daß ein Zwischenträger eingesetzt wird, der durch Auftragen in flüssiger Form vorliegenden Kunststoffes mittels einer oberflächenstrukturierten Vorrichtung auf einen flexiblen Grundkörper hergestellt wird.

3. Verfahren nach Anspruch 2, dadurch gekennzeichnet, daß die oberflächenstrukturierte Vorrichtung eine saugfähige Vorrichtung ist.

4. Verfahren nach Anspruch 2, dadurch gekennzeichnet, daß als oberflächenstrukturierte Vorrichtung eine poröse Vorrichtung, insbesondere eine Schaumstoffrolle oder ein umlaufendes Schaumstoffband verwendet wird.

5. Verfahren nach Anspruch 1, dadurch gekennzeichnet, daß ein Zwischenträger eingesetzt wird, der durch Auftragen in flüssiger Form vorliegenden Kunststoffes auf einen flexiblen Grundkörper hergestellt wird, wobei dem Kunststoff mit einer oberflächenstrukturierten Vorrichtung vor dem Aushärten eine Oberflächenstruktur verliehen wird.

6. Verfahren nach Anspruch 1, dadurch gekennzeichnet, daß ein Zwischenträger eingesetzt wird, der durch Auftragen von Kunststoff nach üblichen Sprühverfahren auf einen flexiblen Grundkörper hergestellt wird, wobei dem Kunststoff mittels einer oberflächenstrukturierten Vorrichtung eine Oberflächenstruktur verliehen wird.

7. Verfahren nach Anspruch 6, dadurch gekennzeichnet, daß als oberflächenstrukturierte Vorrichtung eine saugfähigen Vorrichtung verwendet wird.

8. Verfahren nach Anspruch 6, dadurch gekennzeichnet, daß als oberflächenstrukturierte Vorrichtung eine poröse Vorrichtung, insbesondere eine Schaumstoffrolle oder ein umlaufendes Schaumstoffband verwendet wird.

9. Verwendung eines nach einem Verfahren gemäß einem der Ansprüche 1 - 8 erhaltenen, mit einer mikroporösen Klebstoffschicht beschichteten flexiblen, flächigen Trägers als medizinisches Pflaster.

10. Verfahren zur Herstellung eines Zwischenträgers zur Verwendung in einem Verfahren gemäß Anspruch 1 - 8, dadurch gekennzeichnet, daß ein in flüssiger Form vorliegender, klebstoffabweisender Kunststoff mittels einer oberflächenstrukturierten Vorrichtung, insbesondere einer porösen Vorrichtung, beispielsweise einer Schaumstoffrolle, auf einen flexiblen Grundkörper aufgetragen wird, wobei ein Zwischenträger mit folgenden Eigenschaften erhalten wird:
- er hat eine unter dem Licht- oder Elektronenmikroskop erkennbare gewellte, gefältete, zerklüftete oder gefurchte Oberfläche,
- eine Klebstoffmasse ist von seiner Oberfläche leicht ablösbar, und
- er ist im wesentlichen luftundurchlässig.

11. Verfahren zur Herstellung eines Zwischenträgers zur Verwendung in einem Verfahren gemäß Anspruch 1 - 8, dadurch gekennzeichnet, daß ein in flüssiger Form vorliegender, klebstoffabweisender Kunststoff auf einen flexiblen Grundkörper aufgetragen wird und dem Kunststoff vor dem Aushärten mit einer oberflächenstrukturierten Vorrichtung, insbesondere einer porösen Vorrichtung, beispielsweise einer Schaumstoffrolle, eine Oberflächenstruktur verliehen wird, wobei ein Zwischenträger mit folgenden Eigenschaften erhalten wird:
- er hat eine unter dem Licht- oder Elektronenmikroskop erkennbare gewellte, gefältete, zerklüftete oder gefurchte Oberfläche,
- eine Klebstoffmasse ist von seiner Oberfläche leicht ablösbar, und
- er ist im wesentlichen luftundurchlässig.

## Claims

1. Process for producing a flexible, planar carrier coated with a microporous adhesive layer, characterized in that
a) a flowable adhesive composition is applied onto an intermediate carrier having the following properties:
- it has an undulating, pleated, fissured or furrowed surface detectable under an optical or electron microscope
- the adhesive composition can be easily detached from the surface thereof
- it is essentially air-impermeable,
on application, the adhesive composition not completely filling the depressions of the intermediate carrier,
b) the microscopic air or solvent inclusions resulting, after coating the intermediate carrier, between the adhesive composition and the intermediate carrier are expanded by increasing the temperature until the surface of the adhesive composition bursts, and
c) the adhesive composition is subsequently transferred from the intermediate carrier onto the final carrier.

2. Process according to Claim 1, characterized in that an intermediate carrier is used which is produced by applying plastic in liquid form by means of a surface-structured device onto a flexible basic body.

3. Process according to Claim 2, characterized in that the surface-structured device is an absorbent device.

4. Process according to Claim 2, characterized in that the surface-structured device used is a porous device, in particular a foam roll or a circulating foam belt.

5. Process according to Claim 1, characterized in that an intermediate carrier is used, which is produced by applying plastic in liquid form onto a flexible basic body, the plastic, before curing, being provided with a surface structure by means of a surface-structured device.

6. Process according to Claim 1, characterized in that an intermediate carrier is used, which is produced by applying plastic to a flexible basic body by conventional spraying processes, the plastic being provided with a surface structure by means of a surface-structured device.

7. Process according to Claim 6, characterized in that the surface-structured device used is an absorbent device.

8. Process according to Claim 6, characterized in that the surface-structured device used is a porous device, in particular a foam roll or a circulating foam belt.

9. Use of a flexible, planar carrier obtained by a process according to one of claims 1-8 and coated with a microporous adhesive layer, as a medical plaster.

10. Process for producing an intermediate carrier for use in a process according to Claim 1-8, characterized in that a plastic which is present in liquid form and repels adhesive is applied onto a flexible basic body by means of a surface-structured device, in particular a porous device, for example a foam roll, an intermediate carrier having the following properties being obtained:
- it has an undulating, pleated, fissured or furrowed surface detectable under an optical or electron microscope
- an adhesive composition can be easily detached from the surface thereof, and
- it is essentially air-impermeable.

11. Process for producing an intermediate carrier for use in a process according to Claim 1-8, characterized in that a plastic which is present in liquid form and repels adhesive is applied onto a flexible basic body and the plastic, before curing, is provided with a surface structure by means of a surface-structured device, in particular a porous device, for example a foam roll, an intermediate carrier having the following properties being obtained:
- it has an undulating, pleated, fissured or furrowed surface detectable under an optical or electron microscope
- an adhesive composition can be easily detached from the surface thereof, and
- it is essentially air-impermeable.

## Revendications

1. Procédé de fabrication d'un support plat, souple, enduit d'une couche d'adhésif microporeuse, caractérisé en ce que
a) l'on applique une masse adhésive fluide sur un support intermédiaire qui présente les caractéristiques suivantes :
- il a une surface ondulée, pliée, fissurée ou striée visible au microscope électronique ou optique
- la masse adhésive se décolle facilement de sa surface
- il est essentiellement imperméable à l'air,
la masse adhésive ne remplissant pas complètement les creux du support intermédiaire lors de l'application,
b) les inclusions microscopiques d'air ou de solvant apparaissant après le revêtement du support intermédiaire entre la masse adhésive et le support intermédiaire se dilatent sous l'effet de l'élévation de la température jusqu'à ce que la surface de la masse adhésive éclate, et
c) la masse adhésive est ensuite transférée du support intermédiaire sur le support final.

2. Procédé selon la revendication 1, caractérisé en ce qu'on utilise un support intermédiaire qui est fabriqué par application de plastique se présentant sous forme liquide au moyen d'un dispositif à structure de surface sur un corps de base souple.

3. Procédé selon la revendication 2, caractérisé en ce que le dispositif à structure de surface est un dispositif absorbant.

4. Procédé selon la revendication 2, caractérisé en ce qu'on utilise, en tant que dispositif à structure de surface, un dispositif poreux, en particulier un rouleau de mousse ou une bande de mousse en rotation.

5. Procédé selon la revendication 1, caractérisé en ce qu'on utilise un support intermédiaire qui est fabriqué par application de plastique se présentant sous forme liquide sur un corps de base souple, une structure de surface étant conférée au plastique, avant le durcissage, avec un dispositif à structure de surface.

6. Procédé selon la revendication 1, caractérisé en ce qu'on utilise un support intermédiaire, qui est fabriqué par application de plastique selon des procédés de pulvérisation usuels sur un corps de base souple, une structure de surface étant conférée au plastique au moyen d'un dispositif à structure de surface.

7. Procédé selon la revendication 6, caractérisé en ce qu'on utilise en tant que dispositif à structure de surface un dispositif absorbant.

8. Procédé selon la revendication 6, caractérisé en ce qu'on utilise en tant que dispositif à structure de surface un dispositif poreux, en particulier un rouleau de mousse ou une bande de mousse en rotation.

9. Utilisation d'un support plat, souple, enduit d'une couche d'adhésif microporeuse, obtenu selon un procédé selon l'une quelconque des revendications 1 à 8, en tant que sparadrap médical.

10. Procédé de fabrication d'un support intermédiaire pour l'utilisation dans un procédé selon les revendications 1 à 8, caractérisé en ce que l'on applique un plastique antiadhésif, se présentant sous forme liquide, au moyen d'un dispositif à structure de surface, en particulier un dispositif poreux, par exemple un rouleau de mousse, sur un corps de base souple, un support intermédiaire étant obtenu avec les caractéristiques suivantes :
- il a une surface ondulée, pliée, fissurée ou striée visible au microscope électronique ou optique,
- une masse adhésive se décolle facilement de sa surface, et
- il est essentiellement imperméable à l'air.

11. Procédé de fabrication d'un support intermédiaire pour l'utilisation dans un procédé selon les revendications 1 à 8, caractérisé en ce que l'on applique un plastique antiadhésif, se présentant sous forme liquide, sur un corps de base souple, et que l'on confère une structure de surface au plastique, avant le durcissage, avec un dispositif à structure de surface, en particulier un dispositif poreux, par exemple un rouleau de mousse, un support intermédiaire étant obtenu avec les caractéristiques suivantes :
- il a une surface ondulée, pliée, fissurée ou striée visible au microscope électronique ou optique,
- une masse adhésive se décolle facilement de sa surface, et
- il est essentiellement imperméable à l'air.
